Europäisches Patentamt

European Patent Office

Office européen des brevets

(19)

(11) Publication number: **0 464 163 B1**

(12) **EUROPEAN PATENT SPECIFICATION**

(45) Date of publication of patent specification: **05.04.95**

(51) Int. Cl.⁶: **C08J 9/18**, A61K 9/16, C08L 67/04

(21) Application number: **90916717.3**

(22) Date of filing: **13.11.90**

(86) International application number: **PCT/EP90/01895**

(87) International publication number: **WO 91/09079 (27.06.91 91/14)**

(54) **USE OF SUPERCRITICAL FLUIDS TO OBTAIN POROUS SPONGES OF BIODEGRADABLE POLYMERS.**

(30) Priority: **14.12.89 GB 8928250**

(43) Date of publication of application: **08.01.92 Bulletin 92/02**

(45) Publication of the grant of the patent: **05.04.95 Bulletin 95/14**

(84) Designated Contracting States: **DE GB IT**

(56) References cited:
**EP-A- 0 113 903**
**EP-A- 0 204 476**
**EP-A- 0 251 631**
**US-A- 3 681 270**
**US-A- 4 719 246**

(73) Proprietor: **PHARMACIA S.p.A.**
**Via Robert Koch, 1.2**
**I-20152 Milano (IT)**

(72) Inventor: **DE PONTI, Roberto**
**Via degli Astri, 22**
**I-20147 Milan (IT)**
Inventor: **TORRICELLI, Clara**
**Via M.A. Colonna, 38**
**I-20149 Milan (IT)**
Inventor: **MARTINI, Alessandro**
**Via Tranquillo Cremona, 29**
**I-20145 Milan (IT)**
Inventor: **LARDINI, Ercole**
**Via Rossini, 14A**
**I-20090 Pieve Emanuele (IT)**

(74) Representative: **Ferrario, Vittorino et al**
**Pharmacia/Farmitalia Carlo Erba Srl**
**Patent Department**
**Via Bisceglie, 104**
**I-20152 Milano (IT)**

EP 0 464 163 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention relates to the preparation of porous materials made of biodegradable polymers.

Polymeric matrices which are both porous and biodegradable are useful in a variety of pharmaceutical applications, such as controlled release drug delivery and surgical implantation. For example, a bone graft substitute comprising a biodegradable porous polymer is described in GB-A-2215209, whilst a controlled drug delivery system comprising porous biodegradable polymeric microspheres is discussed by Sato et al in Pharm.Research, Vol.5, No.1, pp 21-30, 1988. A subcutaneously implanted porous polyvinyl alcohol sponge used to treat rats with basic fibroblast growth factor (bFGF) is reported by McGee et al in J.Surgical Research 45, 145-153 (1988), which treatment is observed to accelerate wound healing in the rats.

A convenient new method of making a porous biodegradable polymer has now unexpectedly been found. Accordingly, the present invention provides a method of preparing a biodegradable porous polymer matrix which comprises contacting a biodegradable polymer with a supercritical fluid in a chamber and subsequently reducing the pressure in the chamber to a value below the critical pressure of the fluid in a sharp step.

By supercritical fluid is meant a gas or liquid above its critical point. The critical point of a substance is the point on a state diagram of temperature plotted against pressure at which there ceases to be a dividing line between the gaseous and liquid states. At the critical point physical properties of the liquid and gaseous states, in particular the densities, are identical. The temperature and pressure values at the critical point may be termed the critical conditions and are constant for a given fluid. The critical conditions for a variety of fluids are listed in Table 1 which follows.

TABLE 1

| Critical Conditions for Various Gases and Liquids | | |
|---|---|---|
| | Critical Temperature °C | Critical Pressure kPa (atm) |
| Carbon Dioxide | 31.1 | 7376 (72.8) |
| Ethane | 32.3 | 4884 (48.2) |
| Ethylene | 9.3 | 5036 (49.7) |
| Propane | 96.7 | 4246 (41.9) |
| Propylene | 91.9 | 4620 (45.6) |
| Cyclohexane | 280.3 | 4073 (40.2) |
| Isopropanol | 235.2 | 4762 (47.0) |
| Benzene | 289.0 | 4894 (48.3) |
| Toluene | 318.6 | 4114 (40.6) |
| p-Xylene | 343.1 | 3516 (34.7) |
| Chlorotrifluoromethane | 28.9 | 3921 (38.7) |
| Trichlorofluoromethane | 198.1 | 4408 (43.5) |
| Ammonia | 132.5 | 11280 (111.3) |
| Water | 374.2 | 22050 (217.6) |

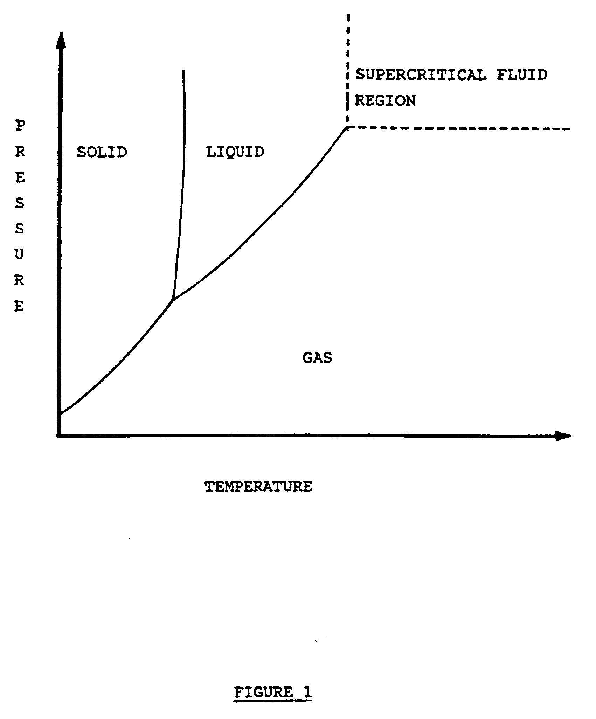

A supercritical region may be defined in the state diagram of a substance, as illustrated in Figure 1 of the accompanying drawings. A fluid is in a supercritical condition when subjected to temperature and pressure values falling within this region.

Any supercritical fluid, including any of those listed above in Table 1, may be used in the process of the present invention. Carbon dioxide, at a pressure of at least 7376 kPa and a temperature of at least 31.1°C, is a preferred example.

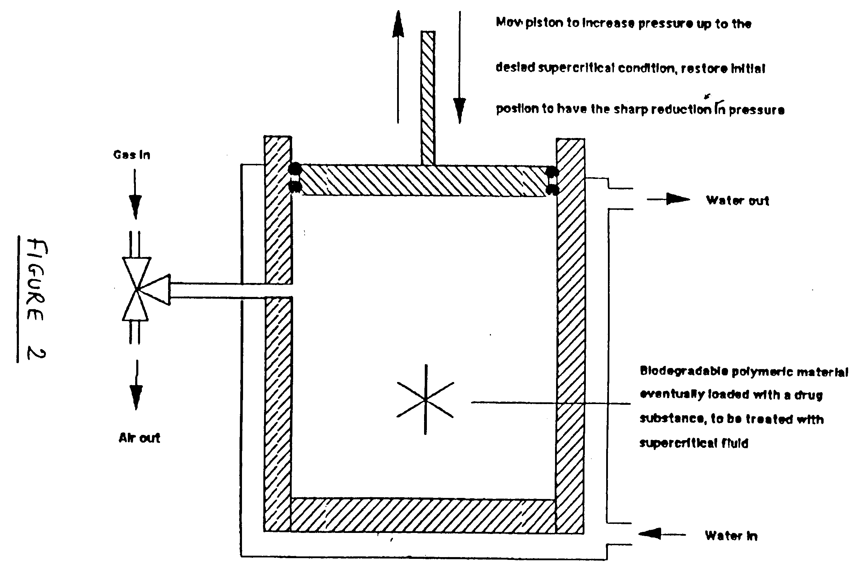

The biodegradable polymer is suitably brought into contact with the supercritical fluid in the chamber of any standard supercritical extractor or any suitable device, examples being the extractor supplied by Muller Extract Company GmbH, the Sample Preparation Accessory supplied by Milton Roy, or the apparatus shown in Figure 2. In operation, the biodegradable polymer is loaded in the extraction chamber of the supercritical extractor and the supercritical fluid is then applied for a time sufficient to allow supercritical fluid to penetrate the mass of the polymer by diffusion, and possibly dissolve the mass. The time needed will vary according to the fluid and the particular supercritical conditions used, and also according to the amount of material to be penetrated or dissolved. The greater the mass of polymer, the longer will be the time needed for the process to be completed, assuming all other parameters controlling the process to remain constant. For a mass of polymer of from 1 to 50g, the time is typically of the order of from 3 minutes to 2 hours. For example, when carbon dioxide is the supercritical fluid it is suitably applied to this mass of material for 45 minutes under conditions of 32°C and 7600 kPa (75 atm), or for 1 hour at 32°C and 15200 kPa (150 atm).

Once the process is complete, the pressure inside the supercritical chamber is reduced in a sharp step. This means that the pressure in the chamber is brought to a value below the critical pressure of the fluid, typically in a period of from 0 to 3 minutes. Preferably the period is from 5 to 30 seconds. The pressure may be reduced in this sharp step to any given value below the critical pressure of the fluid; generally it is between the critical pressure and ambient pressure. If this given value is higher or lower than ambient pressure, the pressure in the chamber is restored to ambient subsequently. In one embodiment of the invention the pressure in the chamber is reduced to ambient pressure in the sharp step. By ambient pressure is meant a pressure of about 101.325 kPa (1 atmosphere).

The sharp pressure reduction contributes towards achieving the desired porosity in the material obtained. The greater the difference between the operating pressure of the supercritical chamber and ambient pressure, the higher is the porosity of the resulting product. The operating temperature of the supercritical chamber can also be adjusted to modify the product porosity; the higher the supercritical temperature employed, the higher is the porosity achieved.

During the process of the invention the operating temperature in the supercritical chamber is controlled. Any suitable control means may be used, for example a water-recirculating bath such as is employed in the Muller extractor, or an air stream such as is employed in the Sample Preparatory Accessory (SPA) apparatus supplied by Milton Roy. The particular operating (supercritical) temperature selected in a given situation will depend partly upon the solubility of the biodegradable polymer in the supercritical fluid since, at a given pressure, this solubility will vary with temperature. It will also partly depend upon the diffusion coefficient of the supercritical fluid in the polymer since, at a given pressure, this coefficient will vary with temperature. As previously indicated, the particular operating temperature employed affects the physical state of the polymer which is achieved.

It is sometimes desirable to operate at the lowest temperature compatible with the solubility of the polymer in the supercritical fluid, and/or with permeation of the supercritical fluid into the polymer under given supercritical pressure conditions. This may be, for example, when a thermally unstable active ingredient is contained in the polymer. At other times, however, this consideration does not apply and a higher operating temperature may be selected.

Any biodegradable polymer may be used as starting material in the process of the invention, suitable examples including polylactides. The term polylactide designates the general class of polymers which can be prepared from one or more of the following monomers: 3-propiolactone tetramethylglycolide, b-butyrolactone, 4-butyrolactone, pivalolactone, and intermolecular cyclic esters of α-hydroxy butyric acid, α-hydroxyisobutyric acid, α-hydroxyvaleric acid, α-hydroxyisovaleric acid, α-hydroxycaproic acid, α-hydroxy-α-ethylbutyric acid, α-hydroxyisopcaproic acid, α-hydroxy-3-methylvaleric acid, α-hydroxyheptanoic acid, α-hydroxyoctanoic acid, α-hydroxydecanoic acid, α-hydroxymyristic acid, α-hydroxystearic acid, and α-hydroxylignoceric acid. It is most preferred to use lactic acid as sole monomer or lactic acid as the principal monomer with glycolic acid as the comonomer. The latter are termed poly(lactide-co-glycolide) copolymers.

Particularly suitable are polymers prepared from lactic acid alone, glycolic acid alone, or lactic acid and glycolic acid wherein the glycolic acid is present as a comonomer in a molar ratio of 100:0 to 40:60. It is most preferred to use a poly(lactide-co-glycolide) copolymer having a molar ratio between about 80:20 and 50:50.

Poly(lactide) homopolymers, poly(glycolide) homopolymers and poly(lactide-co-glycolide) copolymers may range in size from 1,000 to 200,000 in molecular weight stated as an average ($\overline{MW}$). The molecular weight of a particular copolymer is independent of its monomeric makeup. For example, a 50:50 copolymer can have a molecular weight which falls anywhere within this range. Therefore polymers can be varied both as to their monomer composition as well as their molecular weight.

Examples of other suitable biodegradable polymers include: polyanhydrides, for example as described in US-A-4,757,128, Biomaterials 1983, Vol. 4, pages 131-133, J. Biomedical Materials Research, Vol. 19, pages 941-955 (1985), J. Biomedical Materials Research, Vol. 20, pages 51-64 (1986) or Biomaterials 1986, Vol. 7, pages 364-371; and polyacetals, polyketals and poly (orthoesters), for example as described in Polym.Sci.Technol. 1986, Vol. 34, Polymer Med. 2, pages 357-365.

The biodegradable polymeric starting material may be in the form of microspheres, pellets, a matrix of any geometrical shape, or in any other form. When microspheres or any other of the aforementioned starting materials are used these may be, if desired, pre-loaded with an active ingredient so that the process of the invention yields, in one step, a porous spongy material carrying an active ingredient.

Microspheres of a poly(lactide-co-glycolide) copolymer or of a polylactide homopolymer are particularly suitable.

When the biodegradable polymer is in the form of microspheres, these are prepared in a conventional manner. A standard method involves dispersing the biodegradable polymer in an organic solvent, a suitable example being dichloromethane, and adding a silicone oil dropwise to the dispersion with stirring. The microspheres thus obtained are then washed and hardened by treatment with a non-polar hydrocarbon solvent such as pentane or heptane.

The active ingredient contained in the microspheres may be any drug used in surgery, therapy or prophylaxis. Examples include: peptides and proteins, in particular immunomodulators such as Thymic Humoral Factor; growth factors such as basic Fibroblastic Growth Factor, acid Fibroblastic Growth Factor, Epidermal Growth Factor, Human Growth Factor, Insulin Like Growth Factor, Platelet Derived Growth Factor, Nerve Growth Factor and Transforming Growth Factor; antitumorals such as BCNU or 1,3-bis(2-chloroethyl)-1-nitrosourea, daunorubicin, doxorubicin, epirubicin, idarubicin, 4-demethoxydaunorubicin 3'-desamine-3'-(3-cyano-4-morpholinyl) - doxorubicin, 4-demethoxydaunorubicin-3'-desamine-3'-(2-methoxy-4-morpholinyl)-doxorubicin, etoposide and teniposide; hormones such as LHRH and LHRH analogues; and steroideals for birth control and/or antitumoral action such as medroxyprogesterone acetate or megestrol acetate.

If forms of biodegradable polymer other than microspheres are used, such as pellets or matrices of any geometric shape, these may also, if desired, be pre-loaded with an active ingredient such as any of those listed above.

The porous biodegradable polymeric material obtained in accordance with the invention may be used as a surgical implant, for example to aid the reconstitution of tissues after surgical removal of a tumour or after trauma injuries. Such an implant has immediate utility in filling anti-aesthetic voids caused by the surgery or injury, giving support for newly-growing tissue which is able to fill the pores of the material. The biodegradable material itself, as it disintegrates in vivo, will then gradually be replaced by the new tissue. The time required for the degradation of the biodegradable material is related to the composition of the polymer(s), the molecular weight of the polymer, the relative proportions of comonomers (in the case of a copolymer(s)), and the physical state. The process of reconstitution of the damaged tissue is enhanced if the porous polymer is loaded with a suitable growth factor such as any of those listed earlier, chosen according to the particular application.

The porous biodegradable material is also particularly useful in the treatment of tumours which are not removable. In this case the material is loaded with an anti-tumour agent, such as any of those listed earlier, and implanted at the site of the tumour. An example is the treatment of the glioblastoma multiform in the brain.

The porosity (P) of the material produced in accordance with the invention may be calculated, in percentage terms, using the following equation:

$$P = [1-(D1/D2)] \times 100$$

in which D1 is the apparent density of the material and D2 is the true density, measured by the standard method of helium picnometry. A porosity of up to as high as 74% is achieved in accordance with the invention.

Besides giving rise to a highly porous product the process of the invention has the important advantages that, since it allows a non-oxidising agent to be used at a relatively low temperature (e.g. $CO_2$ above 31.1°C), it is relatively non-hazardous and non-toxic and does not cause degradation of any active ingredient contained in the biodegradable polymer starting material. In addition, it is easy to carry out on a large scale and is therefore of economic importance.

The following Examples further illustrate the invention.

Reference Example 1: Preparation of biodegradable microspheres

2g of poly (D,L) lactide, of molecular weight 100,000 and intrinsic viscosity of 1 (Boehringer Ingelheim) were dissolved in 80 ml of $CH_2Cl_2$. 0.8 ml of phosphate buffer (pH 7.4, I = 0.1) were dispersed finely in the $CH_2Cl_2$ phase with an Ultra-Turrax stirring turbine equipment, operating at 4000 rpm with a dispersing tool (type G45F). 60ml of silicone oil Dow Corning Fluid 200 were added dropwise under continued stirring.

The microspheres obtained were washed and hardened with 21 of n-heptane, then desiccated under vacuum at 35°C for 24 hours, and sieved. The microspheres obtained had a mean diameter of about 80 μm.

The n-heptane residual content was about 17%.

Reference Example 2

The process of Reference Example 1 was carried out using poly ((D,L) lactide-co-glycolide) copolymer, having a lactide: glycolide ratio of 75:25, a molecular weight of 18000 and intrinsic viscosity of 0.8 (Boehringer Ingelheim) instead of the poly (D,L) lactide.

The n-heptane residual content was about 10%.

Reference Example 3

The process of Example 2 was repeated using n-pentane in place of n-heptane.

The n-pentane residual content was about 5%

Reference Example 4: Preparation of biodegradable microspheres loaded with an active ingredient

2g of poly (D,L) lactide of molecular weight 100,000 and intrinsic viscosity of 1 (Boehringer Ingelheim) were dissolved in 80ml of $CH_2Cl_2$. 200mcg of basic fibroblast growth factor was dissolved in 0.8ml of phosphate buffer (pH 7.4, I = 0.1) and the aqueous phase dispersed finely in the $CH_2Cl_2$ with an Ultra-Turrax stirring turbine apparatus, operating at 4000 rpm with a dispersing tool (type G45F). 60ml of silicone oil Dow Corning Fluid 200 were added dropwise under continous stirring. The microspheres obtained were washed and hardened with 21 of n-heptane, then desiccated under vacuum at 35°C for 24 hours, and sieved.

The microspheres obtained had a mean diameter of about 80 $\mu$m. The n-heptane residual content was about 17%.

EXAMPLE 1: Preparation of porous biodegradable material

1.5 g of the microspheres obtained by the process of Reference Example 1 were loaded in the extraction chamber of a supercritical extractor (Muller Extract Company GmbH). $CO_2$ was applied at 15200 kPa (150 bar) and 35°C for 1 hour.

The $CO_2$ was taken from a bomb (cylinder) and loaded into the extractor. The pressure of 15200 kPa (150 bar) was applied by means of a mechanical pump. The temperature was kept at 35°C during the process by means of a recirculating water bath. After 1 hour the pressure was reduced to ambient pressure in 10 seconds. The extraction chamber was opened and a spongy material was obtained.

The porosity of the material was determined by the equation:

$$P = [1-(D1/D2)] \times 100$$

where D1 is the apparent density and D2 is the true density measured by helium picnometry and found to be about 76%.

The residual content of n-heptane was 100 ppm.

EXAMPLE 2

The process of Example 1 was repeated but using the following $CO_2$ supercritical conditions: 7600 kPa (75 bar), 32°C, 45 min.

The material obtained was found to have a porosity of about 64%.

EXAMPLE 3

The process of each of Examples 1 and 2 was repeated, but using the biodegradable microspheres loaded with active ingredient prepared in Reference Example 4. A spongy biodegradable materials loaded with the active ingredient was obtained, in each case, having a porosity analogous to that achieved with the unloaded material obtained in Examples 1 and 2.

Reference Example 5

The process of Example 1 was repeated, but using the following $CO_2$ subcritical conditions: 5066 kPa (50 bar), 32°C, 15 min. The material obtained was characterized with a porosity of about 54%.

EP 0 464 163 B1

## Claims

1.  A method of preparing a biodegradable porous matrix which comprises contacting a biodegradable polymer with a supercritical fluid in a chamber and subsequently reducing the pressure in the chamber to a value below the critical pressure of the fluid in a period of from 0 to 3 minutes.

2.  A method according to claim 1 wherein the biodegradable polymer is a polylactide homopolymer or a poly(lactide-co-glycolide) copolymer.

3.  A method according to claim 1 or 2 wherein the biodegradable polymer is in the form of microspheres.

4.  A method according to any one of the preceding claims wherein the biodegradable polymer is loaded with a drug used in surgery, therapy or prophylaxis.

5.  A method according to any one of the preceding claims wherein the supercritical fluid is carbon dioxide at a temperature of at least 31.1 °C and a pressure of at least 7376 kPa .

## Patentansprüche

1.  Verfahren zur Herstellung einer porösen bioabbaubaren Matrix, das den Kontakt eines bioabbaubaren Polymers mit einer superkritischen Flüssigkeit in einer Kammer und das anschliessende Reduzieren des Kammerdrucks auf einen niedrigeren Wert als derjenige des kritischen Drucks der Flüssigkeit innerhalb einer Zeitspanne von 0 bis 3 Minuten umfasst.

2.  Verfahren gemäss Anspruch 1, dadurch gekennzeichnet, dass als bioabbaubares Polymer ein Polylaktid-Homopolymer oder ein Poly(laktid-co-glykolid)co-polymer verwendet wird.

3.  Verfahren gemäss den Ansprüchen 1 oder 2, dadurch gekennzeichnet, dass das bioabbaubare Polymer in Form von Mikrosphären vorliegt.

4.  Verfahren gemäss den obengenannten Ansprüchen, dadurch gekennzeichnet, dass das bioabbaubare Polymer ein in der Chirurgie, Therapie und Prophylaxe angewendetes Arzneimittel enthält.

5.  Verfahren gemäss den obengenannten Ansprüchen, dadurch gekennzeichnet, dass als superkritische Flüssigkeit Kohlendioxyd bei einer Temperatur von mindestens 31.1°C und einem Druck von mindestens 7376 kPa verwendet wird.

## Revendications

1.  Procédé de fabrication d une matrice poreuse biodégradable, comprenant la mise en contact d'un polymère biodégradable avec un fluide supercritique dans une chambre et la réduction successive de la chambre à une valeur inférieure à celle de la pression critique du fluide dans un laps de temps entre 0 et 3 minutes.

2.  Procédé selon la revendication 1, caractérisé par le fait que le polymère biodégradable est un polylactide homopolymère ou un poly(lactide-co-glycolide) copolymère.

3.  Procédé selon la revendication 1 ou 2, caractérisé par le fait que le polymère biodégradable se présente sous forme de microsphères.

4.  Procédé selon l'une des revendications susmentionnées, caractérisé par le fait que le polymère biodégradable contient un médicament employé en chirurgie, thérapie ou prophylaxie.

5.  Procédé selon l'une des revendications susmentionnées, caractérisé par le fait que le fluide supercritique est dioxyde de carbone ayant une température d'au moins 31.1°C et une pressione d'au moins 7276 kPa.

6

SUPERCRITICAL FLUID REGION

PRESSURE

SOLID

LIQUID

GAS

TEMPERATURE

FIGURE 1

FIGURE 2